# EUROPÄISCHE PATENTSCHRIFT

(11) **EP 0 342 392 B1**
(45) Veröffentlichungstag und Bekanntmachung des Hinweises auf die Patenterteilung: **27.10.1993**
(21) Anmeldenummer: 89107407.2
(22) Anmeldetag: 24.04.1989
(51) Int. Cl.: A61N 1/375, B65D 51/00

(54) **Dichtungselement bei einem implantierbaren medizinischen Gerät**
Sealing element for an implantable medical apparatus
Elément d'étanchéité pour appareil médical implantable

(30) Priorität: 16.05.1988 DE 3816640
(43) Veröffentlichungstag der Anmeldung: 23.11.1989
(73) Patentinhaber: Siemens-Elema AB, 171 95 Solna 1 (SE); SIEMENS AKTIENGESELLSCHAFT, 80333 München (DE)
(72) Erfinder: Hirschberg, Jakub, S-18344 Taeby (SE)

(56) Entgegenhaltungen:
- DE-A- 1 450 386
- US-A- 2 264 413
- US-A- 4 240 467
- US-A- 4 479 489

## Beschreibung

Die Erfindung betrifft die Verwendung eines Ringes als Dichtungselement nach dem Oberbegriff des Anspruches 1.

In der US-A-4,141,752 ist ein Herzschrittmacher beschrieben. Zum Befestigen einer Elektrodenleitung in dem Anschlußteil des Herzschrittmachers ist dieses mit einem Loch versehen, in das eine Schraube eingesetzt ist, die das proximale Ende der Elektrodenleitung im Anschlußteil fixiert. Die Schraube ist mit einem elastischen Dichtungselement versehen, das die Öffnung des Loches abdichtet. Zum Drehen der Schraube wird ein Schraubenzieher verwendet, der das Dichtungselement penetriert, um damit die Schraube zu erreichen. Der Nachteil mit einem solchen Dichtungselement besteht darin, daß die Penetrationssstelle danach nicht immer schließt bzw. abdichtet, so daß Körperflüssigkeit in das Anschlußteil eindringen kann. Dies kann zu Leckströmen führen, so daß die Energie der Stimulationsimpulse, die über das distale Ende der Elektrodenleitung einem Patienten zugeführt werden, zum Teil verloren geht und unter Umständen nicht mehr für die Stimulation ausreicht. Durch die Leckströme kann ferner Gewebe im Bereich des Anschlußteils unerwünscht stimuliert werden, so daß Muskelzuckungen entstehen, was für den Patienten unangenehm ist. Durch die Leckströme wird weiterhin die Sensingfunktion der Herzschrittmacherelektrode verschlechtert. Wird das Leck z.B. durch Körperbewegungen geöffnet und geschlossen, kann das vom Herzschrittmacher fälschlich als QRS-Komplex oder P-Welle aufgefaßt werden und unter Umständen zur Inhibierung führen. Bei einem nicht dichten Element kann auch Blut in den Anschluß eindringen und dort koagulieren, so daß es beim Auswechseln eines Herzschrittmachers schwierig ist, die Schraube zu lösen.

In der US-A-4 240 467 ist als Dichtungselement ein Ring der eingangs genannten Art beschrieben, der aber nicht für ein implantierbares medizinisches Gerät vorgesehen, sondern auf einer in einem Ventil angeordneten Welle aufgebracht ist. Der äußere Rand des Ringes ist im Vergleich zum übrigen Ring aus einem härteren Material.

Aus der US-A-2 264 413 ist als Dichtungselemtent ein Y-förmiger Ring aus einem elastischen Material bekannt, der eine Welle eines Gehäuses umschließt.

In der DE-A-1 450 386 ist eine Manschette beschrieben, die zum Abdichten von Rohrenden und Bolzen vorgesehen ist. Die Manschette weist einen Innenring aus verformbarem und federndem Werkstoff auf. Der Innenring ist von einem Außenring aus starrem Material, beispielsweise Metall oder Kunststoff, umgeben.

Der Erfindung liegt die Aufgabe zugrunde, unter Verwendung eines Ringes der eingangs genannten Art ein Dichtungselement zu schaffen, das durchdringbar ist und bei dem gewährleistet ist, daß es immer hermetisch abschließt.

Diese Aufgabe ist erfindungsgemäß durch ein Umkrempeln des Ringes gelöst, so daß dessen äußerer Rand nach innen gekehrt wird und dort die Ringöffnung verschließt. Durch das Umkrempeln des Ringes wird das Dichtungselement bei Betätigung der Schraube mittels eines Werkzeuges nicht penetriert bzw. beschädigt, wobei immer eine gute Abdichtung gegeben ist.

In einer vorteilhaften Ausbildung der Erfindung wird vorgeschlagen, daß der Querschnitt des Ringes nach außen hin abnimmt. Dadurch ist erreicht, daß nach dem Umkrempeln des Ringes die äußere Mantelfläche des Dichtungselementes verhältnismäßig breit ist und damit bei einer Applizierung - z.B. in einem Loch - einen guten Halt ergibt. Gleichzeitig wird damit erreicht, daß der durchdringare Bereich des Dichtungselementes dünn ist.

Die Erfindung ist nachfolgend anhand eines in den Zeichnungen dargestellten Ausführungsbeispieles näher erläutert. es zeigen:
- FIG 1: eine Seitenansicht eines Herzschrittmacheranschlusses mit einem Dichtungselement nach der Erfindung.
- FIG 2 und 3: Seitenansichten eines Dichtungselementes nach FIG 1
- FIG 4 bis 9: Seitenansichten weiterer Ausführungsformen von Dichtungselementen nach der Erfindung.

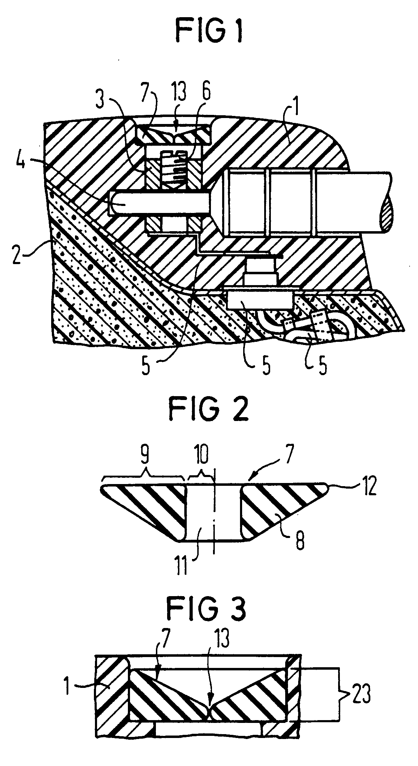

In der FIG 1 ist ein Anschlussteil (1) eines Herzschrittmachers (2) dargestellt. Das Anschlussteil (1) weist eine Verbindungsbuchse (3) auf, in die das proximale Ende (4) einer Elektrodenleitung einführbar ist. Die Verbindungsbuchse (3) dient zur elektrischen Verbindung zwischen dem Herzschrittmacher (2) und der Elektrodenleitung. Diese elektrische Verbindung ist in der FIG 1 mit Bezugszeichen (5) bezeichnet. Das Anschlussteil (1) weist ferner eine Schraube (6) zum elektrischen und mechanischen Verbinden des proximalen Endes (4) der Elektrodenleitung mit der Verbindungsbuchse (3) auf. Zur Abdichtung des Anschlussteiles (1), so dass nach der Implantation des Herzschrittmachers in einen Patienten keine Körperflüssigkeit mit der Verbindungsbuchse bzw. der Schraube in Verbindung kommen kann, ist ein Dichtungselement (7) vorgesehen.

In der FIG 2 ist das Dichtungselement (7) dargestellt. Das Dichtungselement (7), das aus einem elastischen Material hergestellt ist, besteht aus einem Ring (8), dessen Wandstärke (9) grösser ist als der Radius (10) der Ringöffnung (11). Beim Umkrempeln des Ringes (8), so dass dessen äusserer Rand (12) nach innen gekehrt ist, wird - wie in der FIG 3 dargestellt - die Ringöffnung (11) verschlossen. Beim Festschrauben oder Lösen der Schraube (6) kann der Arzt mittels eines nicht dargestellten Schraubenziehers den Mittenbereich (13) des Dichtungselementes (7) durchdringen, ohne das elastische Material zu beschädigen. Nach dem Entfernen des Schraubenziehers vom Dichtungselement (7) schliesst sich der Mittenbereich (13) wieder. Durch das Grössenverhältnis zwischen Wandstärke (9) und Ringöffnung (11) wird der äussere Rand (12) des Ringes (8) mit einem derartig hohen Druck gegeneinander gedrückt, dass das Dichtungselement (7) auch nach Entfernen des Schraubenziehers, hermetisch abschliesst.

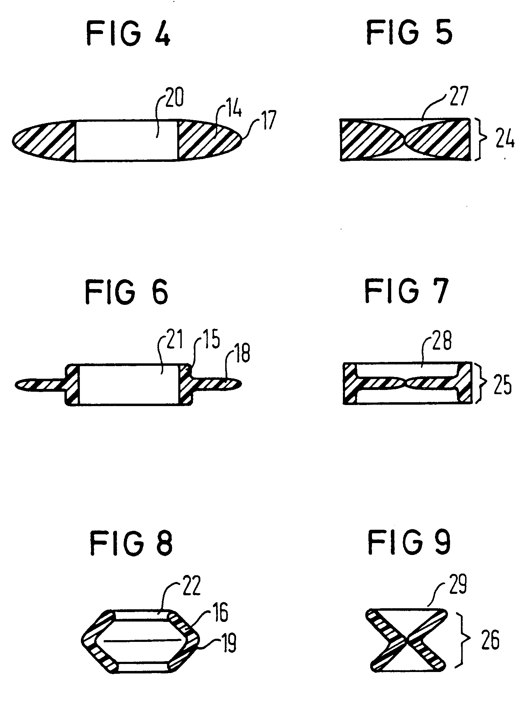

Die FIG 4, 6 und 8 zeigen Ringe (14,15,16) weiterer Dichtungselemente (27,28,29), die verschiedene Ausführungsformen aufweisen. So nimmt der in der FIG 4 gezeigte Ring (14) im Querschnitt nach aussen hin ab. Der Ring in der FIG 5 ist T-förmig und in der FIG 8 V-förmig ausgebildet. In den FIG 5,7 und 9 sind die Ringe (14,15 und 16) umgekrempelt dargestellt, so dass deren äussere Ränder (17,18,19) nach innen gekehrt und die Ringöffnungen (20,21,22) verschlossen sind.

Aus den FIG 2,4,6 und 8 geht hervor, dass der Querschnitt der Ringe (8,14,15,16) nach aussen hin abnimmt. Nach dem Umkrempeln der Ringe (8,14,15,16) sind die äusseren Mantelflächen (23 bis 26) der Dichtungselemente (7,27,28, 29) verhältnismässig breit, so dass diese nach der Applikation in z.B. einem Loch bzw. einer Bohrung gut befestigt werden können. Die Mittenbereiche der Dichtungselemente (7,27,28,29) dagegen sind dünner und somit von einem Schraubenzieher leicht durchdringbar.

Das Dichtungselement nach der Erfindung ist nicht nur für Herzschrittmacher vorgesehen sondern kann mit Vorteil bei weiteren implantierbaren medizinischen Geräten, wie z.B. bei Infusionspumpen verwendet werden.

## Patentansprüche

1. Verwendung eines Ringes aus einem elastischem Material als Dichtungselement bei einem implantierbaren medizinischen Gerät, wobei die Wandstärke des Ringes größer ist als der Radius der Ringöffnung, **gekennzeichnet durch** ein Umkrempeln des Ringes (8,14,15,16) so daß dessen äußerer Rand (12,14,18,19) nach innen gekehrt wird und dort die Ringöffnung (11,20,21,22) verschließt.

2. Verwendung eines Ringes als Dichtungselement nach Anspruch 1, **dadurch gekennzeichnet,** daß der Querschnitt des Ringes (8,14,15,16) nach außen hin abnimmt.

3. Verwendung eines Ringes als Dichtungselement nach Anspruch 1 oder 2, **dadurch gekennzeichnet,** daß der Ring (15) im Querschnitt T-förmig ausgebildet ist.

4. Verwendung eines Ringes als Dichtungselement nach Anspruch 1 oder 2, **dadurch gekennzeichnet,** daß der Ring (16) im Querschnitt V-förmig ausgebildet ist.

## Claims

1. Use of a ring of an elastic material as sealing element for an implantable medical apparatus, in which the wall thickness of the ring is greater than the radius of the ring opening, characterised by a doubling-over of the ring (8, 14, 15, 16) so that its outer rim (12, 14, 18, 19) is turned inwards and there closes the ring opening (11, 20, 21, 22).

2. Use of a ring as sealing element according to Claim 1, characterised in that the cross-section of the ring (8, 14, 15, 16) decreases in an outward direction.

3. Use of a ring as sealing element according to Claim 1 or 2, characterised in that the ring (15) is designed to be T-shaped in cross-section.

4. Use of a ring as sealing element according to Claim 1 or 2, characterised in that the ring (16) is designed to be V-shaped in cross section.

## Revendications

1. Utilisation d'une bague en un matériau élastique en tant qu'élément d'étanchéité dans un appareil médical implantable, l'épaisseur de la paroi de la bague étant supérieure au rayon de l'ouverture de la bague, caractérisé par un retroussement de la bague (8,14,15,16) de sorte que son bord extérieur (12,14,18,19) soit tourné vers l'intérieur et y ferme l'ouverture (11,20,21, 22) de la bague.

2. Utilisation d'une bague en tant qu'élément d'étanchéité selon la revendication 1, caractérisée en ce que la section transversale de la bague (8,14,15,16) diminue vers l'extérieur.

3. Utilisation d'une bague en tant qu'élément d'étanchéité selon la revendication 1 ou 2, caractérisée par le fait que la bague (15) a une section transversale en forme de T.

4. Utilisation d'une bague en tant qu'élément d'étanchéité selon la revendication 1 ou 2, caractérisée en ce que la bague (16) a une section transversale en forme de V.
